# EUROPEAN PATENT APPLICATION

(11) **EP 4 273 878 A1**
(43) Date of publication of application: **08.11.2023**
(21) Application number: 22171638.4
(22) Date of filing: 04.05.2022
(51) Int. Cl.: G16H 40/60

(54) **CONTROLLING LIGHT PROVIDED TO AN INDIVIDUAL**

(71) Applicant: Koninklijke Philips N.V., 5656 AG Eindhoven (NL)
(72) Inventor: KUHLMAN, Daan, Eindhoven (NL); BUIL, Vincentius Paulus, Eindhoven (NL); GEURTS, Lucas Jacobus Franciscus, Eindhoven (NL); LAUTE, Niels, Eindhoven (NL)
(74) Representative: Philips Intellectual Property & Standards

(57) **Abstract**

A mechanism for controlling a size of the pupil(s) of an individual. A current or future task of the individual is used to determine a desired pupil size measurement. The desired pupil size measurement is then used to control or define the operation of one or more light outputting elements and/or light attenuating device, to thereby control the size of the pupil(s).

## Description

### FIELD OF THE INVENTION

The present invention relates to the field of controlling light provided to an individual.

### BACKGROUND OF THE INVENTION

A number of professions require an individual to pay close attention to a screen, display or computer monitor for a significant period of time. For instance, in the medical field, a radiologist tends to work upwards of 40 hours a week during which time they mostly look at screens to analyze displayed medical images.

This need for paying close attention to a screen, display or computer monitor can lead to eyestrain because of the prolonged time spent looking at a screen. This effect can be worsened by poor lighting conditions. For instance, bright overhead light and and/or daylight can force the individual's eyes to strain to see what is on the screen. A bright monitor or bright user interface (UI) elements can also cause the individual's pupils to constrict, giving the eyes a greater range of focus (depth of field).

Another reason for a change in pupil size or diameter is cognitive load of the individual. In particular, there is evidence that there is a pupillary response to performance of a task, and in particular, a mental effort involved in performing a task.

It is known to monitor the size or diameter of an individual's pupil (e.g., using an eye tracking device). With this information it is possible to alter the lighting provided to an individual to improve their working environment.

There is an ongoing desire to improve light provided to an individual.

### SUMMARY OF THE INVENTION

The invention is defined by the claims.

According to examples in accordance with an aspect of the invention, there is provided a computer-implemented method of controlling light provided to an individual by one or more light outputting elements.

The computer-implemented method comprises: obtaining a task indicator, being an indicator of a current task that is being performed by the individual and/or a desired future task to be performed by the individual; determining, responsive to at least the task indicator, an indicator of a desired pupil size measurement; obtaining an indicator of a desired pupil size measurement for the individual; and controlling one or more properties of the one or more light outputting elements and/or one or more light attenuation devices, that controllably attenuate light provided by the one or more light outputting elements, responsive to the indicator of the desired pupil size measurement.

Embodiments of the invention are based on the realization that the diameter of an individual's pupils, i.e. dilation, can be controlled or modified through appropriate of light outputting/attenuation devices. The present disclosure also recognizes that there may be a desire to control the pupil size, e.g., as it has been identified that an individual's ability/capability in performing certain tasks is different for different pupil sizes. For instance, a smaller pupil size is preferred for a radiologist performing an image reading task. Embodiments thereby provide a mechanism for controlling a pupil size to facilitate improved performance of a task.

Thus, rather than reacting to a pupil size to change lighting conditions to suit a current pupil size, the present disclosure proposes to control the light conditions provided to the user to define or control the pupil size. Moreover, proposed embodiments mean that a specific desired pupil size measurement for an individual can be achieved, e.g., rather than a simple modification to increase or reduce pupil size.

A pupil size measurement is any measure of a pupil size, such as a pupil diameter, radius, circumference, area or perimeter, although other suitable measurements of a pupil size will be readily apparent to the skilled person.

The computer-implemented method may further comprise obtaining an indicator of a current pupil size measurement of the individual, wherein the step of controlling the one or more properties of the one or more light outputting elements and/or the one or more light attenuation devices is further responsive to the indicator of the current pupil size measurement.

The current, i.e., present or ongoing, pupil size can thereby be used as a feedback for controlling the pupil size of the subject. In this way, a desired pupil size can be achieved.

In some examples, the step of controlling the one or more properties of the one or more light outputting elements and/or the one or more light attenuation devices comprises: monitoring the current pupil size measurement of the individual using the obtained indicator of current pupil size measurement of the individual; and regulating the one or more properties of the one or more light outputting elements and/or the one or more light attenuation devices until the monitored current pupil size measurement is within a predetermined range of the desired pupil size measurement.

The step of controlling one or more properties of the one or more light outputting elements and/or one or more light attenuation devices may comprise iteratively modifying the one or more properties to transition the pupil size measurement of the individual to the desired pupil size measurement.

This approach provides a mechanism for slowly transitioning the pupils of the individual to a desired pupil size measurement without a sudden or abrupt change. Preferably, at least ten iterative modifications are made, more preferably at least twenty iterative modifications. Such embodiments are particularly advantageous when the task indicator indicates a future task, to gradually accustom the individual to a light level to be used for the future task, i.e., to prepare the individual for the change. Providing a smooth transition prevents or avoids sudden shock or eye strain to a change in light level.

The process of iteratively modifying the one or more properties may comprise performing the iterative modification a predetermined number of times. The predetermined number may be a value no less than ten, e.g., no less than twenty.

In at least one embodiment, the current task is independent of a current view of the individual and/or any desired future task is independent of a view of the individual.

In some embodiments, the individual is a clinician and the current task and/or any desired future task is a clinical task.

In at least one example, the step of determining the indicator of the desired pupil size measurement for the individual comprises: obtaining an indicator of a probable future location of individual; and determining, responsive to at least the task indicator and the probable future location, the indicator of the desired pupil size measurement.

Optionally, the step of determining the indicator of the desired pupil size measurement for the individual comprises: obtaining one or more values for one or more properties of the individual; and determining, responsive to at least the task indicator and the one or more values for the one or more properties of the individual, the indicator of the desired pupil size measurement.

The one or more properties of the individual may comprise one or more of the following: an eye sensitivity of the individual; a medical condition of the individual; and/or a position of the individual with respect to the one or more light outputting elements and/or the one or more light attenuation devices.

The one or more properties of the individual may comprise a predefined preference of the individual.

The computer-implemented method may comprise obtaining an indicator of ambient light conditions in the vicinity of the individual, wherein the step of controlling the one or more properties of the light outputting elements and/or the light attenuation devices is further responsive to the indicator of ambient light conditions in the vicinity of the individual.

The indicator of ambient light conditions may be generated by an ambient light sensing arrangement. Suitable examples may include light-sensitive diodes, light-sensitive resistors or any other form of light-sensitive circuitry, which would be readily apparent to the skilled person.

In some examples, the one or more light outputting elements and/or the one or more light attenuation devices comprises one or more of: a display for providing information to the individual; a light outputting element positioned in glasses or other headwear wearable by the individual; and/or a light attention device positioned in glasses or other headwear wearable by the individual.

The one or more properties of the one or more light outputting elements and/or one or more light attenuation devices may comprise one or more of: for a light outputting element: an intensity of output light; an angle of output light; a color of output light; and/or a beam spread of output light; and/or for a light attenuation device: an amount of attenuated light; and/or a color of attenuated light, for example through electronically controlled electro-chromatic (sun)glasses.

There is also proposed a computer program product comprising computer program code means which, when executed on a computing device having a processing system, cause the processing system to perform all of the steps of any herein described method.

There is also proposed a processing system for controlling light provided to an individual by one or more light outputting elements, the processing system being configured to: obtain a task indicator, being an indicator of a current task that is being performed by the individual and/or a desired future task to be performed by the individual; determine, responsive to at least the task indicator, an indicator of a desired pupil size measurement; and control one or more properties of the one or more light outputting elements and/or one or more light attenuation devices, that controllably attenuate light provided by the one or more light outputting elements, responsive to the indicator of the desired pupil size measurement.

There is also proposed a light control system comprising the processing system and the one or more light outputting elements and/or the one or more light attenuation devices

The one or more light outputting elements and/or the one or more light attenuation devices may comprise one or more of: a display for providing information to the individual; a light outputting element positioned in glasses or other headwear wearable by the individual; and/or a light attention device positioned in glasses or other headwear wearable by the individual.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system comprising a processing system according to an embodiment;
Figure 2 illustrates a method according to an embodiment; and
Figure 3 illustrates a method according to another embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for controlling a size of the pupil(s) of an individual. A current or future task of the individual is used to determine a desired pupil size measurement. The desired pupil size measurement is then used to control or define the operation of one or more light outputting elements and/or light attenuating device, to thereby control the size of the pupil(s).

Embodiments are based on the realization that certain pupil sizes are advantageous for particular tasks. For instance, tasks requiring fine detail (such as analysis of images) would benefit from smaller pupil diameters for improved sensitivity to changes). Tasks that do not require fine detail would benefit from larger pupil diameters, to reduce or minimize eye strain. It would also be beneficial to avoid a sudden or abrupt transition between pupil diameters, to reduce the effect of eye strain. Approaches may be employed in any suitable environment in which an individual performs different tasks that would benefit from different pupil diameters, for instance, clinical, research, domestic and industrial environments.

Embodiments will be described in the context of a clinical environment, in which an individual is a clinician who performs clinical tasks. However, the skilled person will appreciate that embodiments could be expanded to include any other form of environment, e.g., domestic and/or industrial environments, with appropriate characteristics and tasks performed by the individual(s).

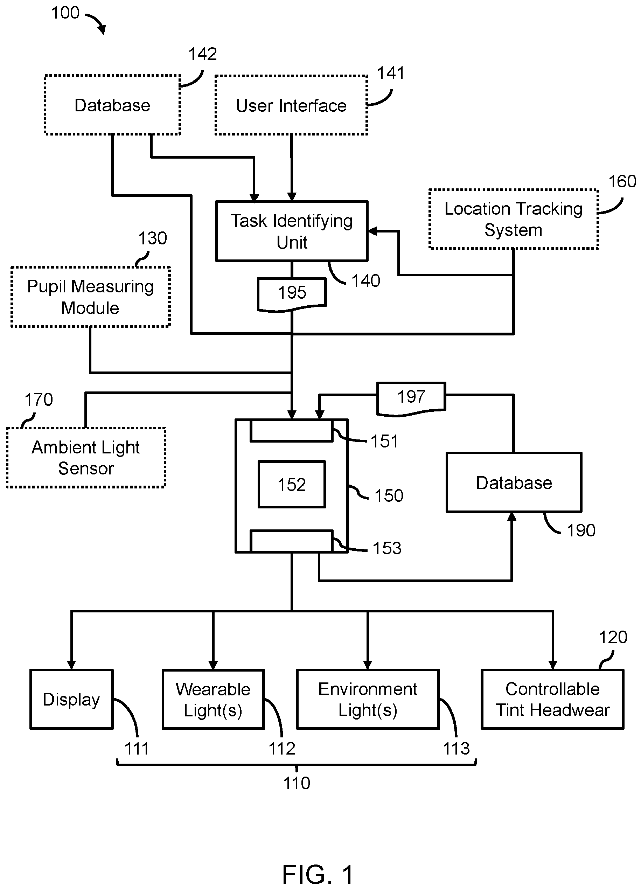

Figure 1 provides an overview of a system 100 according to an embodiment. The system 100 is configured for controlling a pupil dilation of an individual or subject. More specifically, the system is configured for controlling the light incident upon an individual's eyes (or eye) responsive to an indicator of desired pupil size measurement, which is determined based on a current/future task of the individual.

A task is an activity or action taken by the individual in pursuit of a particular purpose. A task may, for instance, be a clinical task (e.g. analyzing and/or assessing medical information and/or treating a subject, e.g. performing a surgical procedure).

Broadly, the system 100 provides a technique for modifying or defining the light intake of an individual's eyes by modifying, affecting or controlling pupil dilation. The system can be used, for instance, to reduce or minimize eye strain of the individual or improve performance of tasks to be carried out by the individual.

The system is adapted to know or identify what task the user is currently working on, or will be working on, and determine a desired pupil dilation based on the requirements or desirable characteristics for this task. The desired pupil dilation may also be dependent upon other data, e.g., one or more of the following: preferences of the individual, ambient lighting conditions, subject information (e.g., color sensitivity, eye size and/or medical conditions of the individual), and position/location of the individual. The system then dynamically modifies pupil dilation of the individual using one or more light emitting/attenuation elements/devices, such as electric darkening glasses (electrochromic glasses); lights in glasses pointed at the user's eyes; environmental lighting (e.g., light bulbs and/or LEDs); user interfaces (e.g., computer screens, smartphone screens, etc.) and similar elements.

The system may also be configured to predict the future lighting need/desire for the individual's eyes. For instance, when the individual moves or changes location, the system may be able to can fluently control the pupil dilation of the individual's eye(s) to adapt to lighting settings of adjacent rooms so the eye stress of going from dark to light environment or vice versa is minimized or reduced.

The system 100 comprises one or more light outputting elements 110 and/or one or more light attenuating elements 120.

A light outputting element is any element, device or system that is able to output light that will be incident upon the individual's eye(s) and which is controllable. Examples include light emitting systems (e.g., bulbs, LEDs and the like), user interfaces (e.g., computer monitors or mobile/cellular phone screens).

In the illustrated example, the one or more light outputting elements comprises a user interface or display 111, one or more wearable lights 112, such as lights mounted/housed in headwear to be worn by the individual, and environmental light(s) 113. Each wearable light, when worn by the individual, is configured to controllably provide and/or direct light into the eye(s) of the individual. Headwear may include glasses and/or other forms of eyewear (e.g., goggles, visors or helmets). Environment lights comprise any light outputting device that changes a light in an environment of the individual, e.g. which may impact on other individuals.

Controlling the properties of any of these elements or devices will control or affect the amount of light incident upon the individual's pupil(s)/eye(s), and therefore pupil dilation. These properties may include, for instance, an intensity and/or color of light emitted. By way of explanation, an increase or decrease in average intensity of light incident upon the individual's eye(s) will respectively decrease or increase a size of the pupil(s) of the individual's eye(s). Similarly, different colors of light will influence the size of pupils upon which the light is incident differently. Without wishing to be bound by theory, it is believed that this is due to the variation in cone sensitivity of the retina of an eye.

Other properties that could be controlled (for a light emitting element) include an angle of output light and/or a beam spread of output light. These characteristics would control or modify an amount of light incident upon the eye(s)/pupil(s) of the individual, and therefore pupil dilation.

A light attenuating element is any element, device or system that is able to reduce or attenuate an amount of light that will be incident upon the individual's pupil(s)/eye(s). In the illustrated example, the one or more light attenuating elements 120 comprises headwear with a (e.g., electrically) controllable level of tint. One example is eyewear having so-called "smart glass" or "switchable glass", whose light transmission properties can be controlled using electricity, light and/or heat. Suitable examples of smart glass include electrochromic and/or thermochromic glass. Other examples of light attenuating elements include light shielding devices, such as controllable blinds or similar structures.

Properties of a light attenuating element that could be controlled using the processing system include an amount of attenuated light; and/or a color of attenuated light, for example through electronically controlled electro-chromatic (sun)glasses. These properties would control or modify an amount and/or color of light incident upon the eye(s)/pupil(s) of the individual.

Thus, each light outputting element and/or light attenuation device is configured to control one or more properties of light, which influence pupil size, incident upon the eye(s) of the individual.

The light outputting element(s) 110 and/or the light attenuating element(s) 120 may be considered to together form a pupil dilation control system, as they facilitate control over the dilation of the pupils.

The operation of the light outputting element(s) 110 and/or the light attenuating element(s) 120 is controlled by a processing system 150, which is itself an embodiment of the invention.

More specifically, the processing system 150 controls one or more properties of the one or more light outputting elements and/or one or more light attenuation devices. Controlling the properties of the light outputting element and/or the one or more light attenuation devices changes or modifies light incident upon the subject to thereby adjust or modify a (actual) pupil measurement of the individual. In this way, the processing system is able to control one or more properties of light incident on the eye(s)/pupil(s) of the individual, and therefore control the pupil size/measurement of the individual.

The properties controlled by the processing system 150 may therefore include any property that affects or influences the size of pupils (e.g., of the eyes) upon which light is incident. This may include an amount of the light, an intensity/luminance of the light and/or the color of the light.

The processing system 150 is configured to determine an indicator 197 of a desired pupil size measurement for the individual. The indicator 197 may be the desired pupil size measurement itself. The indicator 197 is determined using a task indicator 195, which indicates a current task being performed by the individual and/or a desired future task to be performed by the individual. This task indicator 195 is obtained by the processing system 150, e.g., from a task identifying unit 140.

Once the skilled person has appreciated the recognition of this disclosure that a desired pupil size measurement can be derived from a task indicator, approaches that facilitate identification of a desired pupil size measurement using a task indicator and (optionally) one or more other parameters would be readily apparent.

For instance the processing system 197 may be configured to communicate with a database 190 to identify a desired pupil size measurement for a particular current/future task of the individual defined in the task indicator. This may be through the use of a look-up table or the like.

As another example, a current/future task defined in the task indicator may define a range of desired pupil size measurements (e.g., in a look-up table stored by the database 190), and the processing system 150 may be configured to select a desired pupil size measurement within this range, e.g., based on other factors or input indicators as later described.

The desired pupil size measurement(s) and/or ranges, e.g., in the look-up table, may be defined using one or more of the following: population averages (e.g., information derived from literature and/or research), expert analysis (e.g. information provided by experts on a relationship between pupil size and task performance), analysis of historic performance of tasks and associated pupil sizes, and/or feedback from the individual, e.g., via interaction with a user interface.

As yet another example, a formula or weighted algorithm may be used to calculate a desired pupil size measurement based on a task indicator. For instance, each task may be associated with or correspond to a different weight or coefficient value for a formula or weighted algorithm. The weight or coefficient value for the different possible values of the task may be defined by population averages, expert analysis, analysis of historic performance of tasks and associated pupil sizes, and/or feedback from the individual.

As yet another example, an appropriately trained machine-learning algorithm may be used to calculate the desired pupil size measurement. The machine-learning algorithm may receive, as input, the task indicator and provide, as output the desired pupil size measurement. The machine-learning algorithm may be trained using, for instance, appropriately labelled data provided by experts and/or the individual.

The processing system 150 then controls the one or more properties responsive to the indicator 197, i.e., responsive to the desired pupil size measurement. In particular examples, the processing system may be configured to control the one or more light outputting elements and/or one or more light attenuation devices to target a desired pupil size measurement for each individual, i.e. to provide lighting conditions for the individual that is predicted or expected to induce a desired pupil size measurement for that individual.

In particular examples, the processing system may iteratively modifying the one or more properties to transition the pupil size measurement of the individual to the desired pupil size measurement.

Preferably, the processing system may target intermediate pupil size measurements (e.g., between a current pupil size measurement and a desired pupil size measurement) in modifying the pupil size measurement. In other words, each modification performed by the processing system may be configured to control the one or more light outputting elements and/or one or more light attenuation devices to target the intermediate pupil size measurement for each individual, i.e. to provide lighting conditions for the individual that is predicted or expected to induce an intermediate pupil size measurement for that individual.

Thus, each iterative modification (apart from the final modification) may target a different intermediate pupil size measurement. Each intermediate pupil size measurement may lie between a previous intermediate pupil size measurement of the immediately previous modification (or, for the first interaction, a current pupil size measurement or a predicted current pupil size measurement) and a next intermediate pupil size measurement of an immediately next modification (or, for the penultimate modification, the desired pupil size measurement). For the final modification, the desired pupil size measurement may be used instead of the intermediate pupil size measurement.

In some examples, a predetermined period of time is allowed to lapse between each modification, e.g., to allow the individual's eyes time to transition or dilate/constrict. This predetermined period of time may be an average (e.g., global or population average) time for the dilation/constriction of an individual's eyes, and may lie in a region of from 0.5 seconds to 3 seconds. In other examples, as explained below, the size of the pupils is monitored to monitor the effect of the intermediate transition.

The processing system 150 may comprise an input (interface) 151 configured to receive at least the task indicator(s), a data processing unit 152 configured to process at least the task indicator(s) to determine an indicator of desired pupil size measurement, and an output (interface) 153 configured to control one or more properties of one or more light outputting elements 110 and/or one or more light attenuation devices 120 responsive to the indicator of desired pupil size measurement.

The input 151 or input interface may be configured to obtain one or more values for one or more other parameters, and the data processing unit 152 may further use the obtained values in determining the indicator of desired pupil size measurement. Examples of this approach are provided later in this disclosure.

The processing system and the light outputting element(s) 110 and/or the light attenuating element(s) 120 (i.e., the pupil dilation control system) may together form a light control system, according to an embodiment.

The present disclosure thereby envisages an approach in which the light that falls upon a pupil is responsive to a desired pupil size measurement, which is (in turn) based on a current/future task for the individual. It has been recognized that there may be a desire to control the pupil size based on a current/future task, e.g., to achieve an improved or "optimized" pupil size for performing a particular task.

If the task indicator indicates a current task, then the properties may be controlled to target a desired pupil size measurement for the current task.

If the task indicator indicates a future task, then the properties may be controlled to target a desired pupil size measurement for the current task. For instance, the properties may be controlled to gradually change the pupil size measurement up until a time that the future task is performed. Gradually changing the pupil size measurement can be achieved by, for instance, iteratively modifying the one or more properties to effectively transition the pupil size measurement of the individual to the desired pupil size measurement for the future task. Preferably, at least ten iterative modifications are made, more preferably at least twenty iterative modifications.

As a more specific example: if the task indicator includes both a current task and a future task, then the processing system 150 may be configured to identify a desired pupil size measurement for both tasks. During a detected transition from the current task to the future task, the processing system may be configured to control the one or more properties of the light emitting element(s) and/or light attestation device(s) based on interpolated pupil size measurements between the two desired pupil size measurements.

In particular, during a transition from the current task to the future task, the processing system may iteratively modify the one or more properties to transition the pupil size measurement of the individual to the desired pupil size measurement. Preferably, at least ten iterative modifications are made, more preferably at least twenty iterative modifications.

In the context of the present disclosure, a measurement is any measure of a pupil size, such as a pupil diameter, radius, circumference, area or perimeter, although other suitable measurements of a pupil size will be readily apparent to the skilled person. The measurement is a numeric measurement, and is preferably a measurement definable on a continuous scale (such as a distance measurement). The measurement may be a measure of distance, and may be defined in known units of distance such as m, mm, cm, inches and so on.

A variety of approaches or mechanisms for generating the task indicator are envisaged. Any of these approaches may be performed by the task identifying unit 140 of the system 100, e.g. which may provide the task indicator to the processing system 150. The task identifying unit 140 may, in some examples, be formed as part of the processing system 150 itself.

In one example, a user input (received at a user interface 141) may be used to define the task indicator. For instance, a user may be able to input or identify a current task that they are performing, which can be used to define the task indicator.

As another example, the interaction of a user with a user interface 141 or computing system may be monitored to define the task interface. For instance, a task may indicate a current program or application in use by the individual (e.g., to identify whether the user is looking at emails, reviewing patient notes or reviewing medical images). In this way, the task indicator may be inferred from activity of the individual with a computing system or user interface.

As yet another example, an agenda, calendar or schedule of the individual (which may be stored in a database 142) may be used to define a current/future task of the individual. The agenda, calendar or schedule may indicate, for different points in time, the task that is carried out or will be carried out by the individual. A current/future time can be compared to this agenda/calendar/schedule to identify the current/future task of the individual.

As yet another example, a physical position and/or movement of the individual may be tracked and/or analyzed to determine or predict a current/future task of the individual. This may be performed by a location tracking system 160.

Approaches for tracking and/or analyzing the movement of an individual will be readily apparent to the skilled person, and may comprise analyzing one or more images of an environment in which a user is located and/or monitoring trackable elements carried by the individual (e.g., monitoring the location of a mobile/cellular device carried by the individual). Approaches for tracking a location of a subject may include using a camera-based tracking device that monitors the environment of the individual, real-time locating systems, Wi-Fi ranging of computing devices carried by the individual (e.g., wearable devices or mobile/cellular devices). Other approaches would be apparent to the skilled person.

Location information generation by a location tracking system can be used to determine or predict a current task performed, or a future task to be performed, by the individual.

For instance, if the individual is moving towards a computing system, then it can be predicted that the individual will perform a task of using the computing system. As another example, if the individual is moving towards a coffee machine, then it can be predicted that the individual will perform a task of using the coffee machine. As yet another example, different locations or environments may be associated with a limited range or predefined set of one or more tasks. For instance, if a location of an individual is a surgical room, then it can be predicted that the individual is performing a surgical task. Similarly, if a location of an individual is in a surgical preparation room, then it can be predicted that the individual is preparing for surgery (e.g., cleaning).

Location and/or movement of the subject may also be used to detect or predict a transition from a current task to a future task. This information can be used, for instance, to control the one or more properties of the light emitting element(s) and/or light attestation device(s) based on a transition between desired pupil size measurements.

A combination of any of the above-described approaches could be employed. The above-described examples for determining a current/future task of the individual are merely exemplary, and the skilled person would be capable of employing other approaches and/or expanding described approaches for determining the task indicator.

Other parameters, in addition to a current and/or future task, could be used to control or define the desired pupil size measurement and/or the one or more properties of the one or more light outputting elements and/or one or more light attenuation devices. In particular, other parameters may also influence the effect of one or more properties of the one or more light outputting elements and/or one or more light attenuation devices for achieving a desired pupil size measurement.

The system 100 may therefore comprise a number of other optional elements for use in generating and/or defining the desired pupil size measurement and the one or more properties of the one or more light outputting elements and/or one or more light attenuation devices.

The system may therefore comprise a pupil measuring module 130, database 142, a location tracking system 160 and/or an ambient light sensor 170. The data generated by such elements is hereafter described.

For improved control over the pupil size measurement of the individual, in some examples, the processing system 150 is configured to obtain an indicator of a current pupil size measurement of the individual, e.g. from the pupil measuring module 130. The current pupil size measurement may include a pupil size measurement measure of either or both of the pupils of the individual. In one example, if a pupil size measurement is available for both, the current pupil size measurement is an average of the two pupil size measurements.

The processing system may be configured to control the one or more properties of the light outputting elements and/or the light attenuation devices further responsive to (i.e. further dependent upon) the indicator of the current pupil size measurement. Thus, the processing system may effectively regulate the pupil size measurement of the individual.

In other words, the current pupil size measurement may act as a feedback for the processing system targeting the desired pupil size measurement.

The processing system may be configured to control the properties of the light outputting element(s) and/or the light attenuation device(s) to bring the current pupil size measurement within a predetermined range of the desired pupil size measurement. The predetermined range may, for instance, represent an error margin for the current pupil size measurement (e.g., ±10% or ±5% of the desired pupil size measurement). Other suitable ranges would be apparent to the skilled person.

In particular examples, the step of controlling one or more properties of the one or more light outputting elements and/or one or more light attenuation devices may comprise iteratively modifying the one or more properties to transition the pupil size measurement of the individual to the desired pupil size measurement. Each iterative modification may effectively target a respective intermediate pupil size measurement, e.g., between an initial pupil size measurement (being a pupil size before the iterative modifications began) and a desired pupil size measurement. Each intermediate pupil size measurement is a pupil size measurement lying between a previous intermediate pupil size measurement of the immediately previous modification (for all but the first and last iteration(s)) or the initial pupil size measurement (for the first iteration) and the desired pupil size measurement. Of course, the last iteration may target the desired pupil size measurement or a value in a range thereof.

Preferably, at least ten iterative modifications are made, more preferably at least twenty iterative modifications.

The current pupil size measurement can be used to monitor the effect of each iterative modification. In particular, the processing system may maintain a modified one or more properties until the current pupil size measurement reaches an intermediate pupil size measurement (between a previous pupil size measurement for and a desired pupil size measurement) before performing a further modification. The cycle of modifying the properties and monitoring the current pupil size may be repeated until the current pupil size measurement reaches the desired pupil size measurement or is within the predetermined range of the desired pupil size measurement.

The pupil measuring module 130 is configured to determine the current pupil size measurement of the individual. Suitable systems and approaches for determining a current pupil size of the individual would be available to the skilled person.

As a working example, a pupil measuring module 130 may contain one or more sensors that calculate(s) a current pupil size measurement of the individual using the eye's reflection of near infrared light beams and camera tracking. A pupil measuring module 130 may be housed/mounted in headwear, e.g., if present, the same headwear that houses/mounts the one or more light outputting elements and/or one or more light attenuation devices.

Another suitable mechanism for performing pupil size measurement is proposed by US Patent Application having publication number US 2017/0263192 A1, with publication data 14 September 2017 and first named inventor Joaquin F. Luna. Yet another approach usable for determining a pupil size is discussed by US Patent No. US7744216, dated 29 June 2010 with first named inventor Brian L. Uhlhorn.

Other parameters that could be used include: ambient light conditions and/or one or more properties of the subject. Suitable examples for properties of the subject include: location information (e.g. a position of the individual with respect to the one or more light outputting elements and/or the one or more light attenuation devices); and subject information (e.g., an eye sensitivity of the individual; a medical condition of the individual).

The ambient light conditions may be detected by an ambient light sensor 170 of the system 100, which may comprise a camera and/or one or more optical sensors. The subject information may be provided from a database, e.g., the database 142 or the database 190 and/or a user interface. The location information may be obtained from the location tracking system 160.

The skilled person would readily anticipate other suitable parameters that would influence the desired pupil size measurement and/or the effect of one or more properties of the one or more light outputting elements and/or one or more light attenuation devices for achieving a desired pupil size measurement.

In some examples, the processing system uses ambient light conditions to aid in setting or defining the desired pupil size measurement.

This embodiment recognizes that an optimal pupil size measurement for performing a particular task can also depend upon the ambient light conditions. Determining the desired pupil size measurement further based on ambient light conditions thereby facilitates setting of improved light conditions for the subject, so that their pupil size more closely correlates to a desired pupil size for improved performance of the task.

In some examples, the processing system uses ambient light conditions in controlling the one or more properties of the one or more light outputting elements and/or one or more light attenuation devices. For instance, a brightness of ambient light surrounding the individual may be used to define an amplitude of light provided by a light outputting element. As an example, if it is determined that a current pupil size measurement is greater than a desired pupil size measurement, then the brightness of light provided by a light outputting element should be greater than a brightness of ambient light to reduce the pupil size measurement.

As another example, the processing system may further use location information to set or define the desired pupil size measurement. It has previously been explained how location information could be used to predict a current and/or future task, or a transition between tasks, performed by the individual. Approaches for obtaining location information have been previously described.

As yet another example, the processing system may further use subject information to set or define the desired pupil size measurement. It is recognized that the optimal/desired pupil size measurement (for performing a particular task) may vary from individual-to-individual. However, it is anticipated that similar individuals (i.e. individuals having similar subject information) will share similar optimal/desired pupil size measurements for performing a same task, and dissimilar individuals (i.e. individuals having dissimilar subject information) will not share similar optimal/desired pupil size measurements for performing a same task. Use of subject information therefore allows variation in individual's characteristics to be taken into account.

Suitable subject information may include any demographic information (e.g., age, gender etc.), any physical information (e.g., height, weight, eye size), any task familiarity information (e.g., training or experience level in performing the task), any medical history (e.g. medical conditions and the like).

As yet another example, the processing system may further use subject information to control the one or more properties of the one or more light outputting elements and/or one or more light attenuation devices. Some subject information of an individual will directly impact the pupillary response of the individual to changing light conditions.

It is recognized that appropriate lighting settings, for achieving a desired pupil size measurement, will differ depending upon the biological background of the user. For instance, a pupil size of an individual's eye(s) to the same lighting conditions will vary depending upon: age of the individual; sensitivity of an individual's eye(s) to light and/or color and/or a size of the individual's eyes(s). This information is therefore useful for defining suitable lighting conditions for achieving a desired pupil size measurement.

The subject information may, therefore, comprise, age of the individual; sensitivity of an individual's eye(s) to light and/or color and/or a size of the individual's eyes(s). This information can be used to improve the control over the one or more properties of the one or more light outputting elements and/or one or more light attenuation devices.

In some examples, the subject information may indicate one or more predefined preferences of the individual, e.g., pupil size measurements defined or preferred by the individual. A previously described example allows an individual to set or predefine desired pupil size measurements or ranges for different tasks, e.g., through interaction with a user interface.

Once the skilled person has appreciated the recognition of this disclosure that a desired pupil size measurement can be derived from a combination of a task indicator and one or more other parameters, approaches that facilitate identification of a desired pupil size measurement using a task indicator and one or more other parameters would be readily apparent.

For instance, a look-up table may define, for each of a plurality of combinations of values for these parameters, a desired pupil size measurement. Interpolation could be used to identify desired pupil size measurements for combinations of values that are not specifically included in the look-up table.

As yet another example, a formula or weighted algorithm may be used to calculate a desired pupil size measurement based on a task indicator and the one or more other parameters. For instance, each possible value for a task and one or more other parameter may be associated with a different weight or coefficient value for a formula or weighted algorithm. The weight or coefficient value for the different possible values of the task or other parameter may be defined by population averages, expert analysis, analysis of historic performance of tasks and associated pupil sizes, and/or feedback from the individual.

As yet another example, an appropriately trained machine-learning algorithm may be used to calculate the desired pupil size measurement by processing the task and one or more other parameter. The machine-learning algorithm may be trained using appropriately labelled sample data.

Thus, individual-specific information may be used to control the properties of the one or more light outputting elements and/or one or more light attenuation devices.

The processing system 150 may be configured to select which of the one or more light outputting elements and/or one or more light attenuation devices to control based on information of the individual, e.g. location information of the individual.

For instance, a location of the individual may be used to identify those light outputting elements and/or light attenuation devices that will impact the light that falls upon the pupils of the individual, and the processing system 150 may only control the properties of the identified light outputting elements and/or light attenuation devices.

As another example, the location information may be monitored by the processing system to check or identify when/if the individual will change environments (e.g., enter a different environment with different lighting conditions). The processing system may be configured to control the one or more properties of the one or more light outputting elements and/or one or more light attenuation devices to anticipate a change in environment, e.g., controlling a light outputting element in the new environment responsive to the desired pupil size measurement.

This approach can help provide a fluent or smooth transition in lighting so the user can acclimatize to the new environment.

It is also recognized that an individual may not be working or performing a task in a room alone. Thus, multiple individuals may be performing various tasks within a same environment, such that modifying the environmental light conditions for a single individual, responsive to a task said individual is performing, would impact on the pupil size of any other individuals in the same environment. This may affect the efficiency or performance of such other individuals in performing their own tasks.

To at least partially reduce an impact of this approach, the system may be configured to control the one or more light outputting elements and/or one or more light attenuation devices responsive to a plurality of task indicators, each task indicator being associated with a different individual. Thus, each task indicator is an indicator of a current task that is being performed by a particular individual and/or a desired future task to be performed by the particular individual.

The processing system may be configured to control the one or more light outputting elements and/or one or more light attenuation devices to target a desired pupil size measurement for each individual, i.e. provide lighting conditions for each individual that are predicted or expected to induce a desired pupil size measurement for each individual performing their separate tasks.

The precise mechanism employed to achieve this goal depends upon the one or more light outputting elements and/or one or more light attenuation devices available for being controlled by the processing system.

In some examples, each individual is associated with a separate light outputting element, e.g., a different computer monitor, and/or light attenuating device meaning the properties of light incident on the eye(s)/pupil(s) of each individual could be individually controlled responsive to the task indicator for the corresponding individual. In such examples, an ambient light or environmental light property could be controlled to an average light level that provides a best compromise for achieving the desired pupil size measurement for each individual, with further fine-tuning for achieving the desired pupil size measurement achieved using the individual, separate light outputting element and/or light attenuating device.

Although illustrated separately, the database 142 and the database 190 may in fact form one database or may represent a distributed database (e.g., hosted by a cloud-computing system).

Embodiments have been described in the context of a processing system.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

The skilled person would be readily capable of developing a (computer-controlled) method for performing any of the steps carried out by the described processing system, and vice versa.

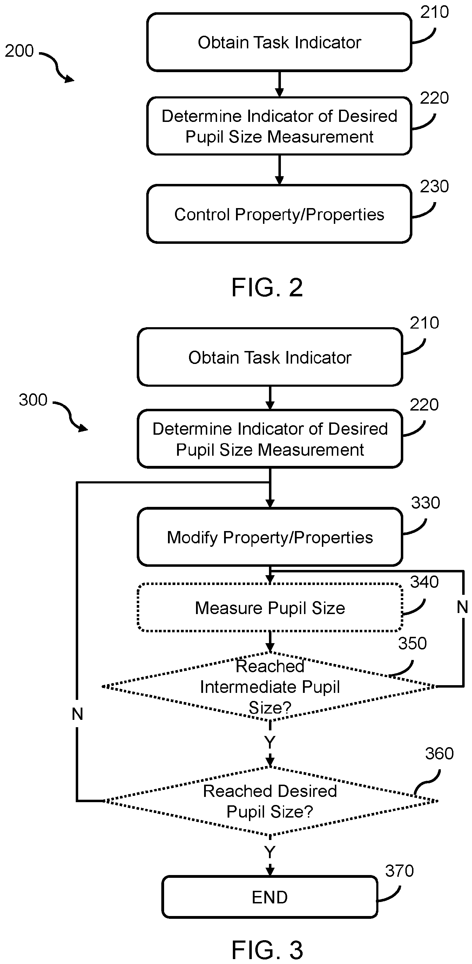

For the sake of completeness, Figure 2 illustrates a method 200 according to an embodiment. The method 200 is for controlling light provided to an individual by one or more light outputting elements.

The method comprises a step 210 of obtaining a task indicator, being an indicator of a current task that is being performed by the individual and/or a desired future task to be performed by the individual.

The method also comprises a step 220 of determining, responsive to at least the task indicator, an indicator of a desired pupil size measurement.

The method also comprises a step 230 of controlling one or more properties of the one or more light outputting elements and/or one or more light attenuation devices, that controllably attenuate light provided by the one or more light outputting elements, responsive to the indicator of the desired pupil size measurement.

Thus, the method proposes an approach of controlling one or more properties of light incident on the eye(s)/pupil(s) of the individual, and therefore controlling the pupil size/measurement of the individual, responsive to a desired pupil size measurement. The desired pupil size measurement is defined by at least a task indicator indicating the current task performed and/or future task to be performed by the individual.

Figure 3 illustrates a method 300 according to another embodiment. The method 300 is for controlling light provided to an individual by one or more light outputting elements.

Like previously described method 200, method 300 comprises a 210 of obtaining a task indicator, being an indicator of a current task that is being performed by the individual and/or a desired future task to be performed by the individual and a step 220 of determining, responsive to at least the task indicator, an indicator of a desired pupil size measurement.

Method 300 differs in comprising a process of iteratively modifying 330 the one or more properties to transition the pupil size measurement of the individual to the desired pupil size measurement. In preferred examples, each iterative modification may target a different intermediate pupil size, excepting the final modification, which targets the desired pupil size measurement.

In some examples, the method 300 comprises, after modifying the one or more properties in step 330, a step 340 of measuring the pupil size.

The measured pupil size may be used as a trigger for performing the next iterative modification. For instance, if the measurement pupil size has reached the intermediate pupil size (as determined in a step 350), then the method may repeat step 330 and further modify the one or more properties. Even when used, it is not essential to use step 350 for the final modification of a modification process (e.g., where a predetermined number of modifications are to be made).

An alternative trigger for performing the next iterative modification after a previous modification is to wait a predetermined period of time after performing a modification before making the next modification. This predetermined period of time may be an average (e.g., global or population average) time for the dilation/constriction of an individual's eyes, and may lie in a region of from 0.5 seconds to 3 seconds, e.g. of from 0.5 seconds to 2 seconds, e.g., 0.5 seconds, 1 second or 2 seconds.

In some examples, the iterative modifications may be performed a predetermined number of times, e.g., under the assumption that by the final modification, the pupil size measurement of the subject will reach the desired pupil size measurement. Thus, in some examples, after before a predetermined number of modifications or iterations of step 330, the method ends in step 370.

As another example, step 330 may be repeated until a current pupil size measurement (determined in a step 360) reaches a desired pupil size measurement. Determination step 360 may determine whether or not the current (determined) pupil size measurement is at the desired pupil size measurement.

If the current pupil size measurement is not equal to the desired pupil size measurement, then modification step 330 can be repeated. Otherwise, the method 300 may end at step 370.

Of course, if step 350 is omitted, then steps 340 and 360 can effectively act as a mechanism for monitoring the current pupil size measurement of the individual and regulating the property/properties until a desired pupil size measurement is achieved.

It will be understood that disclosed methods are preferably computer-implemented methods. As such, there is also proposed the concept of a computer program comprising code means for implementing any described method when said program is run on a processing system, such as a computer.

The computer program may be stored on (tangible) a computer-readable storage medium. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method (200, 300) of controlling light provided to an individual by one or more light outputting elements (110), the computer-implemented method comprising:
obtaining (210) a task indicator (195), being an indicator of a current task that is being performed by the individual and/or a desired future task to be performed by the individual;
determining (220), responsive to at least the task indicator, an indicator (197) of a desired pupil size measurement; and
controlling (230, 330) one or more properties of the one or more light outputting elements and/or one or more light attenuation devices, that controllably attenuate light provided by the one or more light outputting elements, responsive to the indicator of the desired pupil size measurement.

2. The computer-implemented method of claim 1, further comprising obtaining an indicator of a current pupil size measurement of the individual; and
wherein the step of controlling the one or more properties of the one or more light outputting elements and/or the one or more light attenuation devices is further responsive to the indicator of the current pupil size measurement.

3. The computer-implemented method of claim 2, wherein the step of controlling the one or more properties of the one or more light outputting elements and/or the one or more light attenuation devices comprises:
monitoring (330) the current pupil size measurement of the individual using the obtained indicator of current pupil size measurement of the individual; and
regulating (360) the one or more properties of the one or more light outputting elements and/or the one or more light attenuation devices until the monitored current pupil size measurement is within a predetermined range of the desired pupil size measurement.

4. The computer-implemented method of any of claims 1 to 3, wherein the step of controlling one or more properties of the one or more light outputting elements and/or one or more light attenuation devices comprises iteratively modifying the one or more properties to transition the pupil size measurement of the individual to the desired pupil size measurement.

5. The computer-implemented method of any of claims 1 to 4, wherein the current task is independent of a current view of the individual and/or any desired future task is independent of a view of the individual.

6. The method of any of claims 1 to 5, wherein the individual is a clinician and the current task and/or any desired future task is a clinical task.

7. The computer-implemented method of any of claims 1 to 6, wherein the step of determining the indicator of the desired pupil size measurement for the individual comprises:
obtaining an indicator of a probable future location of individual; and
determining, responsive to at least the task indicator and the probable future location, the indicator of the desired pupil size measurement.

8. The computer-implemented method of any of claims 1 to 7, wherein the step of determining the indicator of the desired pupil size measurement for the individual comprises:
obtaining one or more values for one or more properties of the individual; and
determining, responsive to at least the task indicator and the one or more values for the one or more properties of the individual, the indicator of the desired pupil size measurement.

9. The computer-implemented method of claim 8, wherein the one or more properties of the individual comprises one or more of the following: an eye sensitivity of the individual; a medical condition of the individual; and/or a position of the individual with respect to the one or more light outputting elements and/or the one or more light attenuation devices.

10. The computer-implemented method of any of claims 8 or 9, wherein the one or more properties of the individual comprises a predefined preference of the individual.

11. The computer-implemented method of any of claims 1 to 10, further comprising obtaining an indicator of ambient light conditions in the vicinity of the individual; and
wherein the step of controlling the one or more properties of the light outputting elements and/or the light attenuation devices is further responsive to the indicator of ambient light conditions in the vicinity of the individual.

12. The computer-implemented method of any of claims 1 to 11, wherein the one or more properties of the one or more light outputting elements and/or one or more light attenuation devices comprise one or more of:
for a light outputting element: an intensity of output light; an angle of output light; a color of output light; and/or a beam spread of output light; and/or
for a light attenuation device: an amount of attenuated light; and/or a color of attenuated light.

13. A computer program product comprising computer program code means which, when executed on a computing device having a processing system, cause the processing system to perform all of the steps of the method according to any of claims 1 to 12.

14. A processing system (150) for controlling light provided to an individual by one or more light outputting elements (110), the processing system being configured to:
obtain (210) a task indicator (195), being an indicator of a current task that is being performed by the individual and/or a desired future task to be performed by the individual;
determine (220), responsive to at least the task indicator, an indicator of a desired pupil size measurement; and
control (230, 330) one or more properties of the one or more light outputting elements and/or one or more light attenuation devices (120), that controllably attenuate light provided by the one or more light outputting elements, responsive to the indicator of the desired pupil size measurement.

15. A light control system (100) comprising the processing system of claim 14 and the one or more light outputting elements (110) and/or the one or more light attenuation devices (120).
